# EUROPEAN PATENT APPLICATION

(11) **EP 2 479 190 A1**
(43) Date of publication of application: **25.07.2012**
(21) Application number: 10817241.2
(22) Date of filing: 16.09.2010
(51) Int. Cl.: C07K 16/18, C12Q 1/37, G01N 33/53, G01N 33/577

(54) **COLLAGEN NEOEPITOPE ANTIBODY**

(30) Priority: 16.09.2009 JP 2009213962
(71) Applicant: Shionogi Co., Ltd., Osaka-shi, Osaka 541-0045 (JP)
(72) Inventor: NUMATA, Yoshito, Toyonaka-shi Osaka 561-0825 (JP); YAMAUCHI, Akira, Toyonaka-shi Osaka 561-0825 (JP); ONODA, Junji, Toyonaka-shi Osaka 561-0825 (JP); YAMANE, Shoji, Toyonaka-shi Osaka 561-0825 (JP); MAEDA, Tomoko, Toyonaka-shi Osaka 561-0825 (JP)
(74) Representative: Denison, Christopher Marcus
(86) International application number: PCT/JP2010/066029
(87) International publication number: WO 2011/034128

(57) **Abstract**

The present invention provides a novel monoclonal antibody that is specific for a C-terminal neoepitope of a collagen fragment and has substantially equal binding affinity whether proline contained in the neoepitope is in a non-hydroxylated form or in a hydroxylated form, and an immunoassay, a measurement method, a kit and the like using the antibody. The antibody allows for quantification of a collagen fragment generated by digestion of a biological sample with a collagenase present in the sample, regardless of the presence or absence of a hydroxylated form of proline in the neoepitope.

SEQUENCE LISTING

<110>  Shionogi&Co.,LTD

<120>  A collagen neoepitope antibody

<130>  10P00078WO

<160>  20

<170>  PatentIn version 3.1

<210>  1
<211>  7
<212>  PRT
<213>  Artificial

<220>
<223>  collagen neopeptide

<400>  1

Gly Pro Pro Gly Pro Gln Gly
1               5

<210>  2
<211>  7
<212>  PRT
<213>  Artificial

<220>
<223>  collagen neopeptide

<220>
<221>  misc_feature
<222>  (3)..(3)
<223>  Hydroxyproline

<400>  2

Gly Pro Xaa Gly Pro Gln Gly
1               5

<210>  3
<211>  141
<212>  PRT
<213>  Artificial

<220>
<223>  VH of Antibody 20A10

<400>  3

Phe Leu Met Ala Ala Ala Gln Gly Ile Gln Ala Gln Ile Gln Leu Val
1               5                   10                  15

Gln Ser Gly Pro Glu Leu Lys Glu Pro Gly Glu Thr Val Arg Ile Ser
            20                  25                  30

Cys Lys Ala Ser Gly Tyr Thr Phe Thr Lys Tyr Gly Ile Asn Trp Val

35                  40                  45

Lys Gln Ala Pro Gly Lys Gly Leu Glu Trp Met Ala Trp Ile Asn Thr
    50                  55                  60

Tyr Ser Gly Met Thr Thr Tyr Ala Asp Asp Phe Lys Gly Arg Phe Ala
65                  70                  75                  80

Phe Ser Leu Glu Thr Ser Ala Asn Thr Ala Tyr Leu Gln Ile Asn His
                85                  90                  95

Leu Lys Asn Asp Asp Thr Ala Thr Tyr Phe Cys Ala Arg Ser Leu Gly
            100                 105                 110

Tyr Asp Tyr Gly Gly Phe Ala Tyr Trp Gly Gln Gly Thr Leu Val Thr
        115                 120                 125

Val Ser Ala Ala Lys Thr Thr Pro Pro Ser Val Tyr Pro
    130                 135                 140

<210>  4
<211>  125
<212>  PRT
<213>  Artificial

<220>
<223>  VL of Antibody 20A10

<400>  4

Trp Ile Pro Val Ser Ser Ser Asp Val Leu Leu Thr Gln Thr Pro Leu
1               5                   10                  15

Ser Leu Pro Val Ser Leu Gly Asp Gln Ala Phe Ile Ser Cys Arg Ser
            20                  25                  30

Gly Gln Thr Leu Val His Asp Asn Glu Asn Thr Tyr Phe His Trp Tyr
        35                  40                  45

Leu Gln Lys Pro Gly Gln Ser Pro Lys Leu Leu Ile Tyr Lys Ile Ser
    50                  55                  60

Asn Arg Phe Ser Gly Val Pro Asp Arg Phe Ser Gly Ser Gly Ser Gly
65                  70                  75                  80

Thr Asp Phe Thr Leu Lys Ile Ser Arg Val Glu Pro Glu Asp Leu Gly
                85                  90                  95

Ile Tyr Phe Cys Ser Gln Asn Thr His Val Pro Phe Thr Phe Gly Ser
            100                 105                 110

Gly Thr Lys Leu Glu Ile Lys Arg Ala Asp Ala Ala Pro
        115                 120                 125

<210>  5
<211>  5
<212>  PRT
<213>  Artificial

<220>
<223>  CDR1 of 20A10 heavy chain

<400>  5

Lys Tyr Gly Ile Asn
1               5

<210>  6
<211>  17
<212>  PRT
<213>  Artificial

<220>
<223>  CDR2 of 20A10 heavy chain

<400>  6

Trp Ile Asn Thr Tyr Ser Gly Met Thr Thr Tyr Ala Asp Asp Phe Lys
1               5                   10                  15

Gly

<210>  7
<211>  11
<212>  PRT
<213>  Artificial

<220>
<223>  CDR3 of 20A10 heavy chain

<400>  7

Ser Leu Gly Tyr Asp Tyr Gly Gly Phe Ala Tyr
1               5                   10

<210>  8
<211>  16
<212>  PRT
<213>  Artificial

<220>
<223>  CDR1 of 20A10 light chain

<400>  8

Arg Ser Gly Gln Thr Leu Val His Asp Asn Glu Asn Thr Tyr Phe His
1               5                   10                  15

<210>  9

<211>  7
<212>  PRT
<213>  Artificial

<220>
<223>  CDR2 of 20A10 light chain

<400>  9

Lys Ile Ser Asn Arg Phe Ser
1               5


<210>  10
<211>  9
<212>  PRT
<213>  Artificial

<220>
<223>  CDR3 of 20A10 light chain

<400>  10

Ser Gln Asn Thr His Val Pro Phe Thr
1               5


<210>  11
<211>  9
<212>  PRT
<213>  Artificial

<220>
<223>  collagen type 1 specific seaquence

<400>  11

Gly Ser Pro Gly Ala Asn Gly Pro Ala
1               5


<210>  12
<211>  9
<212>  PRT
<213>  Artificial

<220>
<223>  collagen type 2 specific seaquence

<400>  12

Gly Gln Pro Gly Asp Asp Gly Pro Ser
1               5


<210>  13
<211>  9
<212>  PRT
<213>  Artificial

<220>
<223>  collagen type 3 specific seaquence

<400>  13

Gly Gln Leu Gly Ser Pro Gly Ala Gln
1               5

<210>  14
<211>  14
<212>  PRT
<213>  Artificial

<220>
<223>  neoepitope peptide

<220>
<221>  MISC_FEATURE
<222>  (10)..(10)
<223>  Hydroxyproline

<400>  14

Asp Gly Pro Ser Gly Ala Gln Gly Pro Xaa Gly Pro Gln Gly
1               5                   10

<210>  15
<211>  4
<212>  PRT
<213>  Artificial

<220>
<223>  neoepitope peptide

<400>  15

Gly Pro Gln Gly
1

<210>  16
<211>  12
<212>  PRT
<213>  Artificial

<220>
<223>  neoepitope peptide

<400>  16

Gly Pro Pro Gly Pro Gln Gly Leu Ala Gly Gln Arg
1               5                   10

<210>  17
<211>  19
<212>  PRT
<213>  Artificial

<220>
<223>  neoepitope peptide

<220>
<221>  MISC_FEATURE
<222>  (15)..(15)
<223>  Hydroxyproline

<400>  17

Gly Gln Pro Gly Ala Ala Gly Pro Ser Gly Ala Gln Gly Pro Xaa Gly
1               5                   10                  15

Pro Gln Gly

<210>  18
<211>  19
<212>  PRT
<213>  Artificial

<220>
<223>  neoepitope peptide

<400>  18

Gly Gln Pro Gly Ala Ala Gly Pro Ser Gly Ala Gln Gly Pro Pro Gly
1               5                   10                  15

Pro Gln Gly

<210>  19
<211>  22
<212>  PRT
<213>  Artificial

<220>
<223>  collagen neoepitope

<220>
<221>  MISC_FEATURE
<222>  (18)..(18)
<223>  Hydroxyproline

<400>  19

Gly Gln Leu Gly Gln Pro Gly Ala Ala Gly Pro Ser Gly Ala Gln Gly
1               5                   10                  15

Pro Xaa Gly Pro Gln Gly
            20

<210>  20
<211>  975
<212>  PRT
<213>  Homo sapiens

<400>  20

Met Ile Arg Leu Gly Ala Pro Gln Thr Leu Val Leu Leu Thr Leu Leu
1               5                   10                  15

Val Ala Ala Val Leu Arg Cys Gln Gly Gln Asp Val Gln Glu Ala Gly

            20                  25                  30

Ser Cys Val Gln Asp Gly Gln Arg Tyr Asn Asp Lys Asp Val Trp Lys
        35                  40                  45

Pro Glu Pro Cys Arg Ile Cys Val Cys Asp Thr Gly Thr Val Leu Cys
    50                  55                  60

Asp Asp Ile Ile Cys Glu Asp Val Lys Asp Cys Leu Ser Pro Glu Ile
65                  70                  75                  80

Pro Phe Gly Glu Cys Cys Pro Ile Cys Pro Thr Asp Leu Ala Thr Ala
                85                  90                  95

Ser Gly Gln Pro Gly Pro Lys Gly Gln Lys Gly Glu Pro Gly Asp Ile
            100                 105                 110

Lys Asp Ile Val Gly Pro Lys Gly Pro Pro Gly Pro Gln Gly Pro Ala
        115                 120                 125

Gly Glu Gln Gly Pro Arg Gly Asp Arg Gly Asp Lys Gly Glu Lys Gly
    130                 135                 140

Ala Pro Gly Pro Arg Gly Arg Asp Gly Glu Pro Gly Thr Pro Gly Asn
145                 150                 155                 160

Pro Gly Pro Pro Gly Pro Pro Gly Pro Pro Gly Pro Pro Gly Leu Gly
                165                 170                 175

Gly Asn Phe Ala Ala Gln Met Ala Gly Gly Phe Asp Glu Lys Ala Gly
            180                 185                 190

Gly Ala Gln Leu Gly Val Met Gln Gly Pro Met Gly Pro Met Gly Pro
        195                 200                 205

Arg Gly Pro Pro Gly Pro Ala Gly Ala Pro Gly Pro Gln Gly Phe Gln
    210                 215                 220

Gly Asn Pro Gly Glu Pro Gly Glu Pro Gly Val Ser Gly Pro Met Gly
225                 230                 235                 240

Pro Arg Gly Pro Pro Gly Pro Pro Gly Lys Pro Gly Asp Asp Gly Glu
                245                 250                 255

Ala Gly Lys Pro Gly Lys Ala Gly Glu Arg Gly Pro Pro Gly Pro Gln
            260                 265                 270

Gly Ala Arg Gly Phe Pro Gly Thr Pro Gly Leu Pro Gly Val Lys Gly
        275                 280                 285

His Arg Gly Tyr Pro Gly Leu Asp Gly Ala Lys Gly Glu Ala Gly Ala
    290                 295                 300

Pro Gly Val Lys Gly Glu Ser Gly Ser Pro Gly Glu Asn Gly Ser Pro
305                 310                 315                 320

Gly Pro Met Gly Pro Arg Gly Leu Pro Gly Glu Arg Gly Arg Thr Gly
                325                 330                 335

Pro Ala Gly Ala Ala Gly Ala Arg Gly Asn Asp Gly Gln Pro Gly Pro
            340                 345                 350

Ala Gly Pro Pro Gly Pro Val Gly Pro Ala Gly Gly Pro Gly Phe Pro
        355                 360                 365

Gly Ala Pro Gly Ala Lys Gly Glu Ala Gly Pro Thr Gly Ala Arg Gly
    370                 375                 380

Pro Glu Gly Ala Gln Gly Pro Arg Gly Glu Pro Gly Thr Pro Gly Ser
385                 390                 395                 400

Pro Gly Pro Ala Gly Ala Ser Gly Asn Pro Gly Thr Asp Gly Ile Pro
                405                 410                 415

Gly Ala Lys Gly Ser Ala Gly Ala Pro Gly Ile Ala Gly Ala Pro Gly
            420                 425                 430

Phe Pro Gly Pro Arg Gly Pro Pro Gly Pro Gln Gly Ala Thr Gly Pro
        435                 440                 445

Leu Gly Pro Lys Gly Gln Thr Gly Glu Pro Gly Ile Ala Gly Phe Lys
    450                 455                 460

Gly Glu Gln Gly Pro Lys Gly Glu Pro Gly Pro Ala Gly Pro Gln Gly
465                 470                 475                 480

Ala Pro Gly Pro Ala Gly Glu Glu Gly Lys Arg Gly Ala Arg Gly Glu
                485                 490                 495

Pro Gly Gly Val Gly Pro Ile Gly Pro Pro Gly Glu Arg Gly Ala Pro
            500                 505                 510

Gly Asn Arg Gly Phe Pro Gly Gln Asp Gly Leu Ala Gly Pro Lys Gly
        515                 520                 525

Ala Pro Gly Glu Arg Gly Pro Ser Gly Leu Ala Gly Pro Lys Gly Ala
    530                 535                 540

Asn Gly Asp Pro Gly Arg Pro Gly Glu Pro Gly Leu Pro Gly Ala Arg
545                 550                 555                 560

Gly Leu Thr Gly Arg Pro Gly Asp Ala Gly Pro Gln Gly Lys Val Gly
                565                 570                 575

Pro Ser Gly Ala Pro Gly Glu Asp Gly Arg Pro Gly Pro Pro Gly Pro
            580                 585                 590

Gln Gly Ala Arg Gly Gln Pro Gly Val Met Gly Phe Pro Gly Pro Lys
        595                 600                 605

Gly Ala Asn Gly Glu Pro Gly Lys Ala Gly Glu Lys Gly Leu Pro Gly
    610                 615                 620

Ala Pro Gly Leu Arg Gly Leu Pro Gly Lys Asp Gly Glu Thr Gly Ala
625                 630                 635                 640

Ala Gly Pro Pro Gly Pro Ala Gly Pro Ala Gly Glu Arg Gly Glu Gln
                645                 650                 655

Gly Ala Pro Gly Pro Ser Gly Phe Gln Gly Leu Pro Gly Pro Pro Gly
            660                 665                 670

Pro Pro Gly Glu Gly Gly Lys Pro Gly Asp Gln Gly Val Pro Gly Glu
        675                 680                 685

Ala Gly Ala Pro Gly Leu Val Gly Pro Arg Gly Glu Arg Gly Phe Pro
    690                 695                 700

Gly Glu Arg Gly Ser Pro Gly Ala Gln Gly Leu Gln Gly Pro Arg Gly
705                 710                 715                 720

Leu Pro Gly Thr Pro Gly Thr Asp Gly Pro Lys Gly Ala Ser Gly Pro
                725                 730                 735

Ala Gly Pro Pro Gly Ala Gln Gly Pro Pro Gly Leu Gln Gly Met Pro
            740                 745                 750

Gly Glu Arg Gly Ala Ala Gly Ile Ala Gly Pro Lys Gly Asp Arg Gly
        755                 760                 765

Asp Val Gly Glu Lys Gly Pro Glu Gly Ala Pro Gly Lys Asp Gly Gly
    770                 775                 780

Arg Gly Leu Thr Gly Pro Ile Gly Pro Pro Gly Pro Ala Gly Ala Asn
785                 790                 795                 800

Gly Glu Lys Gly Glu Val Gly Pro Pro Gly Pro Ala Gly Ser Ala Gly
                805                 810                 815

Ala Arg Gly Ala Pro Gly Glu Arg Gly Glu Thr Gly Pro Pro Gly Pro
            820                 825                 830

Ala Gly Phe Ala Gly Pro Pro Gly Ala Asp Gly Gln Pro Gly Ala Lys
        835                 840                 845

Gly Glu Gln Gly Glu Ala Gly Gln Lys Gly Asp Ala Gly Ala Pro Gly
    850                 855                 860

Pro Gln Gly Pro Ser Gly Ala Pro Gly Pro Gln Gly Pro Thr Gly Val
865                 870                 875                 880

Thr Gly Pro Lys Gly Ala Arg Gly Ala Gln Gly Pro Pro Gly Ala Thr
                885                 890                 895

Gly Phe Pro Gly Ala Ala Gly Arg Val Gly Pro Pro Gly Ser Asn Gly
            900                 905                 910

Asn Pro Gly Pro Pro Gly Pro Pro Gly Pro Ser Gly Lys Asp Gly Pro
        915                 920                 925

Lys Gly Ala Arg Gly Asp Ser Gly Pro Pro Gly Arg Ala Gly Glu Pro
    930                 935                 940

Gly Leu Gln Gly Pro Ala Gly Pro Pro Gly Glu Lys Gly Glu Pro Gly
945                 950                 955                 960

Asp Asp Gly Pro Ser Gly Ala Glu Gly Pro Pro Gly Pro Gln Gly
                965                 970                 975

## Description

### Technical Field

The present invention relates to a monoclonal antibody capable of recognizing a neoepitope of collagen, and an immunoassay, a measurement method, screening, patient selection, and a kit based thereon.

### Background Art

Cartilage degradation is a major feature of joint diseases, such as osteoarthritis (OA) and rheumatoid arthritis (RA). In such diseases, monitoring of progression of cartilage loss provides useful information for disease management (including prognosis, diagnosis, and treatment). At present, cartilage loss is monitored by detecting narrowing of the joint space by X-ray. However, X-ray diagnosis is far from satisfactory in terms of sensitivity and accuracy of detection. X-ray diagnosis merely indicates past occurrence of cartilage loss and not the current state of cartilage degradation. Accordingly, alternative methods for monitoring progression of cartilage destruction in various joint diseases will be very valuable.

To date it has been revealed that the degradation of collagen in cartilage is carried out by a group of enzymes called matrix metalloproteinases (MMPs). In particular, in view of the fact that only collagenases (MMP-1, MMP-8, and MMP-13) are responsible for cleavage of the triple helix of native type II collagen, cleavage between the amino acid residues at positions 975 and 976 of type II collagen by the collagenases is considered as a key event that leads to subsequent removal of type II collagen from the cartilage matrix by proteases, such as gelatinases. As used herein, the amino acid residue refers to the full length sequence of COL2A1 deposited the GenBank database (Accession Number: NP001835).

The fragments of degraded extracellular matrix proteins are released from the cartilage into the synovial fluid (SF) and then into systemic circulation through the bloodstream. Thus, detection of the serum or urine levels of the fragments of cartilage matrix proteins, if possible, will allow for simpler and more convenient assessment of cartilage degradation in OA and RA, although they are generally lower than the SF levels.

There are two major problems for measuring the systemic levels of type II collagen fragments. The first problem is that turnover of type II collagen in the articular cartilage is normally very low and thus the serum or urine levels of type II collagen fragments are also very low. Although there is an increase in the turnover of type II collagen in the OA cartilage, it is not a sufficiently large change that would dramatically facilitate the detection. Therefore, highly sensitive assays are required.

The second problem is that a large number of the proline and lysine residues that constitute collagen are hydroxylated. In particular, since proline is a major component of the collagen proteins that account for about 30% of collagen protein, and hydroxylated prolines are found in the vicinity of collagenase cleavage sites, hydroxylation of proline is known to largely affect the binding affinity.

Since hydroxylation of the prolines present in collagen is catalyzed by prolyl 4-hydroxylase in an iron- and vitamin C-dependent manner, the degree of the hydroxylation is readily influenced by conditions, such as nutritional status, at the collagen synthesis, and it is not constant. Therefore, antibodies used for measurement of type II collagen fragments are desirably those whose binding affinity is not affected by the presence or absence of the proline hydroxylation.

To date, several systems for measuring type II collagen fragments generated by collagenases have been invented; however, none of them is sufficient in sensitivity and accuracy. For example, in the competitive ELISA method of Robin Poole *et al*. with the monoclonal antibody COL2-3/4C long (Patent Document 1), the specificity to the structure of the collagenase-cleaved end is low, and further it has almost no reactivity with the unhydroxylated form of proline at position 5 upstream from the cleavage site (at position 971 from the N-terminus) (Non-Patent Document 1). With respect to the sandwich ELISA method of Otterness *et al*. using the monoclonal antibody 9A4 (Patent Document 2), it shows high specificity to the structure of the collagenase-cleaved end; however, the binding affinity is reduced to about 1/90th by hydroxylation of proline at position 5 upstream from the cleavage site (at position 971 from the N-terminus) (Non-Patent Document 2). Thus, the detection sensitivity is reduced for type II collagen fragments majorly hydroxylated at this position.

Considering that, as described above, the antibodies hitherto used are all prone to be affected by hydroxylation of proline in the immediate vicinity of the collagenase cleavage site, it should be appreciated that accurate measurement of the amount of collagen fragments present in a biological sample which is a mixture of the hydroxylated and non-hydroxylated forms has been impossible.

### Prior Art References

### Patent Documents

Patent Document 1: Japanese Patent No. 2999416
Patent Document 2: Japanese Patent No. 3258630

### Non-Patent Documents

Non-Patent Document 1: J. Immunol. Methods, 294, pp. 145-153 (2004)
Non-Patent Document 2:J. Immunol. Methods, 247, pp. 25-34 (2001)

### Summary of the Invention

### Problem to be Solved by the Invention

An object of the present invention is to provide accurate measurement of the amount of a collagen fragment (a collagen neoepitope) generated by collagenase digestion in biological samples.

### Means for Solving the Problem

The present inventors have made extensive studies for generating a monoclonal antibody against a neoepitope of collagen. As a result, the present inventors have completed the present invention by generating a novel monoclonal antibody whose binding capacity is not altered when proline in the neoepitope is converted into a hydroxylated form.

Thus, the present invention relates to:
(1) A monoclonal antibody specifically binding to a collagen neoepitope fragment at positions 962 to 975 of the amino acid sequence shown in SEQ ID NO: 20, wherein the binding affinity is substantially the same whether proline contained in the epitope is in a non-hydroxylated form or in a hydroxylated form;
(2) The monoclonal antibody described in (1), wherein the proline described in (1) is located at position 971 of the amino acid sequence shown in SEQ ID NO: 20;
(3) The monoclonal antibody described in (1), wherein the epitope is located in the region of positions 957 to 975 of the amino acid sequence shown in SEQ ID NO: 20;
(4) The monoclonal antibody described in (2), wherein in an immunoassay using the peptide consisting of the amino acid sequence shown in SEQ ID NO: 14, 17, or 18 as the competitor to inhibit the immunoreaction of the antibody with the peptide consisting of the amino acid sequence shown in SEQ ID NO: 2, 50% inhibitory concentration for the immunoreaction is 0.04 µM or lower for either of the peptides;
(5) A monoclonal antibody having:
   1) in a complementarity-determining region, a heavy chain variable region containing the following amino acid sequences: KYGIN (SEQ ID NO: 5), WINTYSGMTT YADDFKG (SEQ ID NO: 6), and SLGYDYGGFAY (SEQ ID NO: 7); and
   2) in a complementarity-determining region, a light chain variable region containing the following amino acid sequences: RSGQTLVHDNENTYFH (SEQ ID NO: 8), KISNRFS (SEQ ID NO: 9), and SQNTHVPFT (SEQ ID NO: 10);
(6) A monoclonal antibody having:
   1) a heavy chain variable region containing the amino acid sequence of SEQ ID NO: 3; and
   2) a light chain variable region containing the amino acid sequence of SEQ ID NO: 4;
(7) The monoclonal antibody described in any one of (1) to (6), which is labeled;
(8) An immunoassay using the monoclonal antibody described in any one of (1) to (6);
(9) A method for measuring the amount of a collagen neoepitope fragment, using the monoclonal antibody described in any one of (1) to (6);
(10) A method for measuring collagenase activity, wherein the amount of a collagen neoepitope fragment measured using the monoclonal antibody described in any one of (1) to (6) is used as an indicator;
(11) A method for screening for a collagenase inhibitor, wherein the amount of a collagen neoepitope fragment measured using the monoclonal antibody described in any one of (1) to (6) is used as an indicator;
(12) A method for selecting patients with collagenase related diseases, comprising a step of measuring the amount of a collagen neoepitope fragment contained in a biological sample, by using the monoclonal antibody described in any one of (1) to (6);
(13) A kit comprising the monoclonal antibody described in any one of (1) to (6);
(14) A method for diagnosing collagenase-related diseases, comprising a step of measuring the amount of a collagen neoepitope fragment contained in a biological sample, by using the monoclonal antibody described in any one of (1) to (6); and
(15) The monoclonal antibody described in any one of (1) to (6) for use in diagnosis of collagenase-related diseases.

### Effect of the Invention

Since the monoclonal antibody of the present invention is capable of specifically recognizing the terminal neoepitope structure and is not affected by hydroxylation of proline, it allows for accurate detection and quantification of the amount of a collagen neoepitope fragment in organisms.

### Brief Description of the Drawings

Fig. 1 shows the collagenase cleavage site at the 2/3 position from the amino-terminus in the schematic diagram of triple helical trimeric fibrillar collagen (types I, II, and III). In the lower part, the collagenase cleavage site and adjacent amino acid sequences of type II collagens from various animals. From top to bottom, human, bovine, canine, rat, and murine sequences are shown, and correspond to positions 954-980 of human type II collagen. The asterisk indicates the cleavage site, which is located between amino acid residues 975 and 976.
Fig. 2 shows the results of competitive immunoassays with peptide fragments having different C-terminal structures of 20A10.
Fig. 3 shows, in the upper, the results of competitive immunoassays for 20A10 with collagenase-digested collagen and non-digested collagen. The table shown in the lower part of the figure indicates the changes in the specificity by collagenase-digestion and the cross-reactivity between the fragments of types I, II, and III.
Fig. 4 shows the results of sandwich immunoassays using a combination with a type II collagen-specific monoclonal antibody.
Fig. 5 shows the measurements of the concentrations of a collagen neoepitope fragment in the degradation of type II collagen by MMP-13, added at a concentration of 1.2 ng/well, with addition of various concentrations of an MMP inhibitor.
Fig. 6 shows the measurements of the concentrations of a collagen neoepitope fragment in bovine cartilage explant culture in the presence of 1 ng/ml human interleukin 1.
Fig. 7 shows the amino acid sequences of the variable regions of 20A10. The upper and lower parts show the variable regions of the heavy and light chains, respectively. The underlined sequences in each sequence indicate the locations of the complementarity-determining regions.

### Mode for Carrying out the Invention

### I. Antibody

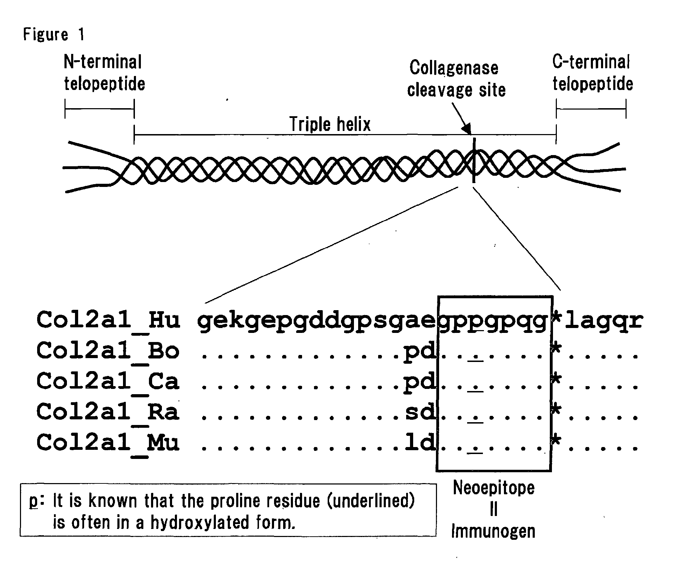

Fibrillar collagens, types I, II, and III collagens, are composed of three peptide chains coiled together into a helical form. Collagenases (e.g., MMP-1, MMP-8, andMMP-13) cleave the triple helix of these collagens at a site three quarters of the distance from their N-terminus (between the amino acid residues at positions 975 and 976) in the native form. The terminal structures given rise to the C-terminus of the N-terminal three-quarter fragment and the N-terminus of the C-terminal quarter fragment are referred to as "neoepitopes" (Fig. 1). The collagenase fragments generated by the cleavage are referred to as "collagen neoepitope fragments." Among other fragments, the seven amino acid residues (Gly-Pro-Pro-Gly-Pro-Gln-Gly (SEQ ID NO: 1)) of the neoepitope region at the C-terminus of human type II collagen (Accession No. NP_001835) (corresponding to positions 969-975; hereinafter referred to as C-terminal neoepitope) are known to be conserved among animal species including humans and mice. Collagen is a protein characterized by a high hydroxyproline content, and the proline residue at position 5 from the C-terminus (or at position 971 from the N-terminus) of its sequence is often in a hydroxylated form (hereinafter referred to as hydroxylated proline or hydroxyproline) (SEQ ID NO: 2, Gly-Pro-Hyp-Gly-Pro-Gln -Gly, in which Hyp represents hydroxyproline). The percentage of hydroxylation is not constant, as it is readily influenced by conditions, such as health status and nutritional status, at the collagen synthesis, and such conditions are considered to hamper accurate qualification of collagen neoepitope fragments. Thus, provided that collagen degradation is measured by immunological means, it is intrinsically desirable for an antibody detecting a neoepitope to have the property that can recognize both hydroxylated and non-hydroxylated forms (of proline) to the same degree in an immunologically specific manner.

The monoclonal antibody of the present invention is characterized in that the binding affinity is not altered when proline contained in the type II collagen neoepitope fragment (SEQ ID NO: 20), which has a neoepitope at the C-terminus, is converted into a hydroxylated form. As used herein, the phrase "the binding affinity is not altered" means that the binding affinity is substantially the same whether proline of the amino acid residues constituting the neoepitope is proline (non-hydroxylated form) or hydroxyproline (hydroxylated form).

The term "binding affinity" generally refers to the strength or affinity of a type of noncovalent interaction between an immunoglobulin molecule and an antigen specific for the immunoglobulin; and may often be expressed as the dissociation constant (Kd).

The term "substantially the same" specifically means that the binding affinity of a hydroxylated form (hydroxyproline) is within the range of 80% to 120%, preferably within the range of 90% to 110%, and more preferably within the range of 95% to 105% of the binding affinity value of the non-hydroxylated form. The term also means that the cross-reactivity between non-hydroxylated (proline) and hydroxylated forms is 80% or more, preferably 90% or more, and more preferably 95% or more. The binding affinity of an antibody may be determined by a known method, for example, Scatchard analysis with ELISA assays (e.g., Campbell, 1991; and Segel, 1976).

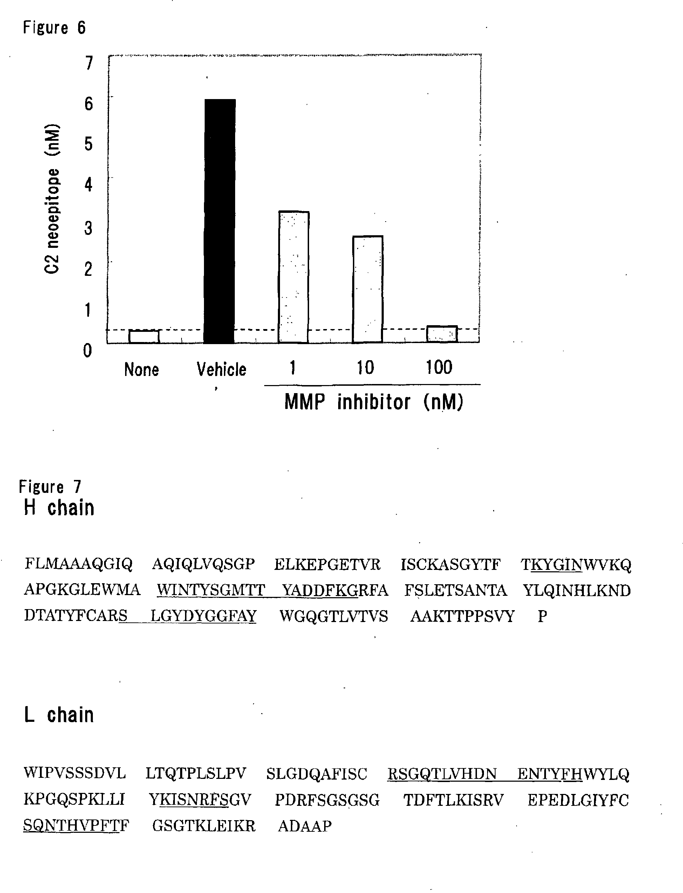

A representative example of such a monoclonal antibody may be 20A10. The amino acid sequences of the variable regions of 20A10 are shown in Fig. 6. The upper part shows the sequence of the heavy chain (SEQ ID NO: 3) and the lower part shows that of the light chain (SEQ ID NO: 4). The underlined parts indicate the locations of the complementarity-determining regions (CDRs) (SEQ ID NOs: 5-10).

The immunogen used for generation of the monoclonal antibody of the present invention may be prepared using a method as described, for example, in Antibodies:A Laboratory Manual (1989, Cold Spring Harbor Laboratory Press).

Immunization may be performed using a conventional method, for example, by administering the immunogen to mammals by injection, such as intravenous, intradermal, subcutaneous, or intraperitoneal injection. More specifically, for example, the immunogen is diluted to a suitable concentration with, for example, physiological saline-containing phosphate buffer (PBS) or a physiological saline solution, and administered to test animals several times at intervals of 2-3 weeks in combination, if desired, with a conventional adjuvant. When mice are used, the dose per administration is approximately 50-100 µg for each mouse. As used herein, the adjuvant refers to a substance that enhances the immune response in a non-specific manner when administered in combination with the antigen. Conventionally used adjuvants include, for example, pertussis vaccines and Freund's adjuvant. An antiserum may be obtained by drawing blood from a mammalian animal 3-10 days after the final immunization.

A method for produce a monoclonal antibody may be carried out by preparing fusion cells (hybridomas) between plasma cells from mammals immunized with the immunogen (immune cells) and mammalian plasmacytoma cells (myeloma cells), selecting, from these hybridomas, a clone that produces a desired monoclonal antibody that recognizes 5'-deoxy-5'-methylthioadenosine, and then culturing the clone. Basically, the production of the monoclonal antibody may be conducted in accordance a conventional method.

In the method, the mammals to be immunized with the immunogen are desirably selected in consideration of the compatibility with the plasmacytoma cells used for cell fusion; mice, rats and the like are used. The immunization method is the same as that used for preparation of polyclonal antibodies. However, spleen cells are removed from the immunized animals 3-10 days after the final immunization.

To obtain hybridomas from the immune cells thus obtained, a method described in "Experimental Manual for Molecular Cell Biology" (Takekazu Horie et al., published in 1994, Nankodo) may be used. In order to form cells that can be passaged by subculture, plasmacytoma cells are fused with the antibody-producing immune cells; for example, in the presence of sendaivirus or polyethylene glycol, whereby hybridomas may be obtained. The plasmacytoma cells used here are desirably plasmacytoma cells derived from a homothermal animal of the same species; for example, when fused with spleen cells obtained using mice as immunized animals, mouse myeloma cells are preferably used. Known cells, such as p3x63-Ag8.UI, may be used as the plasmacytoma cells.

Hybridomas are selected with HAT medium (supplemented with hypoxanthine, aminopterin, and thymidine). Once emergence of colonies is observed, the antibodies secreted into the culture supernatant are tested (screened) for the binding to the antigen, whereby a hybridoma that produces an antibody of interest may be obtained.

The screening methods include various methods generally used for detection of antibodies, for example, the spot test, the agglutination reaction test, Western-blotting, and ELISA. Preferably, as detailed below, the screening method is carried out according to the ELISA method on the hybridoma culture supernatant, using the reactivity with the neoepitope peptide as an indicator. By this screening, it is possible to screen for an isolate that produces an antibody of interest that is specifically reactive with the neoepitope peptide. Clone 20A10 is an example of the clones obtained based on this process.

Cloning of the isolates obtained as a result of the screening which are capable of producing antibodies of interest may be carried out by a conventional method, such as limiting dilution analysis or soft agar analysis. The cloned hybridomas may be cultured in a large scale, if necessary, either in serum-containing or serum-free medium. By this culture, it is possible to obtain the desired antibody with a relatively high purity. Alternatively, it is possible to inoculate the hybridomas into the abdominal cavity of mammals, such as mice, that are compatible with the hybridomas to recover the desired antibody in large quantity as mouse ascites fluid.

The culture supernatant and mouse ascites fluid that contain the hybridoma that produces the monoclonal antibody of the present invention may be used as a crude antibody solution without purification or modification. Isolation/purification of the monoclonal antibody may be carried out by subjecting the culture supernatant or the ascites fluid to saturated ammonium sulfate, ion exchange chromatography (e.g., DEAE or DE52), or affinity column chromatography, such as anti-immunoglobulin column or protein A column chromatography.

Alternatively, a recombinant antibody produced using a genetic recombination technique by cloning an antibody gene, inserting it into an appropriate vector, and introducing the vector into a host may be used as the monoclonal antibody of the present invention (for example, Carl et al., THERAPEUTIC MONOCLONAL ANTIBODIES, published in 1990).

Specifically, cDNAs encoding the variable regions (for example, SEQ ID NOs: 3 and 4 from 20A10) of an antibody of interest (for example, 20A10), are synthesized. For synthesis and amplification of the cDNAs, 5'-Ampli FINDER RACEKit (Clonetech) and the 5'-RACE method using PCR (Frohman, M. A. et al, Proc. Natl. Acad. Sci. USA 1988, vol. 85, p. 8998) may be available. DNA fragments of interest are purified from the obtained PCR products and ligated to vector DNA. Further, desired recombinant vectors are prepared by introducing recombinant vectors into a host such as E. coli, and selecting colonies. The nucleotide sequences of the DNAs of interest are confirmed by a known method, such as the dideoxy method.

Once the DNAs encoding the V regions of the antibody of interest have been obtained, they are ligated to DNA encoding the desired antibody constant region (C region) and integrated into expression vectors. Alternatively, the DNAs encoding the V regions of the antibody may be integrated into expression vectors containing DNA encoding the antibody C region. In order to produce the antibody for use in the present invention, the antibody gene is integrated into an expression vector so as to be expressed under the control of an expression regulatory region, for example, under the control of an enhancer/promoter. Then, host cells may be transformed with the expression vector to express the antibody.

Expression of the antibody gene can be achieved either by cotransformation of a host with expression vectors into which the heavy chain (H chain) and light chain (L chain) of the antibody are separately integrated, or by transformation of a host with a single expression vector into which DNA encoding both the H and L chains is integrated (see WO94/11523).

In performing immunoassays (immunological measurements) as described below using an antibody, in general, the antibody *per se* may be labeled with various substances so that the behavior of the antibody can be detected. Preferred embodiments of the monoclonal antibody of the present invention include a labeled antibody. Labeling of the antibody may be conducted according to a conventional method, such as described, for example, in "Experimental Manual for Molecular Cell Biology" (Takekazu Horie et al., 1994, Nankodo). The various substances include chemiluminescent substances, enzymes, fluorescent substances, colored beads, radioisotopes, elements, metals, and biotin. Specific examples include, but are not limited to, the following: chemiluminescent substances such as luminol and acridinium esters; enzymes such as β-galactosidase, alkali phosphatase, and peroxidase; fluorescent substances such as europium cryptate, FITC, and RITC; colored beads such as Protein A beads, wheat germ agglutinin (WGA) beads, streptavidin beads; radioisotopes such as ¹⁴C, ¹²⁵I, and ³H; elements such as lanthanides, such as europium; and metals such as ferritin and gold colloids.

### II. Immunoassay

The monoclonal antibody of the present invention is capable of specifically recognizing the C-terminal neoepitope structure of a collagen neoepitope fragment. This neoepitope does not depend on the types of collagen. Thus, the monoclonal antibody allows for quantification of the collagen neoepitope fragment for any collagens by performing sandwich assays in combination with antibodies capable of recognizing epitopes specific for each collagen (see above for the preparation process). The specific sequences for each type of collagen are well known to one skilled in the art; exemplary sequences are as follows:
Type I collagen specific sequence:
   Gly-Ser-Pro-Gly-Ala-Asp-Gly-Pro-Ala (SEQ ID NO: 11)
Type II collagen specific sequence:
   Gly-Glu-Pro-Gly-Asp-Asp-Gly-Pro-Ser (SEQ ID NO: 12)
Type III collagen specific sequence:
   Gly-Glu-Lys-Gly-Ser-Pro-Gly-Ala-Gln (SEQ ID NO: 13)

The monoclonal antibody of the present invention, either labeled or unlabeled, is useful for immunoassays (immunological measurements). Immunoassays using the monoclonal antibody of the present invention may be competitive or non-competitive. The phrase "50% inhibitory concentration for the immunoreaction of an antibody is 0.04 µM or lower" means that rate of inhibition of the binding between the peptide consisting of the amino acid sequence shown in SEQ ID NO: 2 and the antibody is 50% or more when, for example, the peptide consisting of the amino acid sequence shown in SEQ ID NO: 14, 17, or 18 is added at a concentration of 0.04 µM (Example 2). The 50% inhibitory concentration is preferably 0.04 µM or lower, and more preferably 0.022 µM or lower. The immunoassays may be either homogeneous assays (measurements by a homogeneous system) or heterogeneous assays (measurements by a heterogeneous system). Specifically, examples include enzyme immunoassays (EIA), enzyme-linked immunosorbent assays (ELISA), fluoroimmunoassays (FIA), radioimmunoassays (RIA), time-resolved fluoroimmunoassays (TR-FIA), chemiluminescent immunoassays, immunoblotting, Western blotting, immunostaining, SPA methods, fluorescence polarization (FP), and fluorescence resonance energy transfer (FRET).

Preferred embodiments of the immunoassay of the present invention include ELISA methods. ELISA method refers to a method using enzyme-labeled antibodies or antigens for quantifying antibodies or antigens by measuring the activity of the labeling enzyme. This method uses an enzyme-labeled antigen-antibody complex and free enzyme-labeled antigen, or solid-phased antibody or antigen for separation of antibody. Substances such as agarose, the inside surface of microtiter plates, or latex particles may be used as the solid phase. Specifically, the ELISA methods may be competitive immunoassays, sandwich immunoassays, and the like. The labeling enzymes may be horseradish peroxidase (hereinafter referred to as HRP), alkali phosphatase, and the like.

### III. Utility

The monoclonal antibody and immunoassay of the present invention are useful in various applications. For example, the monoclonal antibody and immunoassay of the present invention are useful for the activity determination of collagenases, such as MMPs. Three members of collagenase are known to be capable of cleaving the triple helix of intact fibrillar collagen, i.e., collagen types I, II, and III (Pendas AM et al., Genomics (1995) 26: 615-8, and Mitchell PG et al., J Clin Invest (1996) 97: 761-8). Since the monoclonal antibody of the present invention is capable of specifically recognizing a neoepitope fragment generated by collagenase cleavage, measurement of the amount of the fragment is possible, which allows for estimation of collagenase activity.

The monoclonal antibody and immunoassay of the present invention are also useful in screening methods which use the amount of a collagen neoepitope as an indicator. In such screening, recombinant collagenase (in a purified or partially purified form) prepared using, for example, an expression vector, is maintained in the presence of a test substance under conditions (for example, in 0.1 M phosphate buffer, pH 7.4, at room temperature) that allow binding of the enzyme to its substrate (collagen), and the test substance is examined to determine whether it can inhibit the binding of the enzyme's substrate; thus, the collagen neoepitope fragment is qualified. In this process, the test substance may be any of the following: a peptide, protein, non-peptidic compound, synthetic compound (low molecular weight compound), fermented product, cell extract, plant extract, and animal tissue extract. Also, the test substance may be a sample containing these substances.

Candidate substances selected as collagenase inhibitors by the screening may be potential prophylactic or therapeutic agents for diseases (for example, osteoarthritis, proliferative diseases, including cancer, osteoporosis, Alzheimer's disease, and hypertension) to which collagenase is known to be related.

The monoclonal antibody and immunoassay of the present invention are useful for patient selection, comprising the step of measuring the amount of a collagen neoepitope fragment contained in a biological sample. For example, the immunoassay of the present invention allows for measurement of the collagen epitope fragment amount in a biological sample from a patient (any biological fluid samples generally tested in clinical sampling may be used including, for example, a body fluid, such as blood, urine, saliva, and sweat; and an extract or supernatant from cells and/or tissue). Such biological fluid samples are safely obtained without any risk, and measurements of such samples are easy and inexpensive. Thus, for diseases (for example, osteoporosis, osteoarthritis, rheumatoid arthritis, and other diseases causing benign or malignant bone tumors or cartilage destruction) whose progression is indexed by the collagen neoepitope fragment amount, routine and mass screening of such diseases are possible. Further, the monoclonal antibody and immunoassay of the present invention may be used to determine the degree of ongoing collagen destruction in patients with different types of osteoarthritis or rheumatoid arthritis.

The kit of the present invention is characterized by containing the monoclonal antibody of the present invention as a binding agent for the detection of a collagen neoepitope fragment present in a test sample. In general, such a kit further comprises one or more components necessary to carry out assays. Such components may be reference standards, reagents (diluents and buffers and the like), containers, and/or devices. For example, a container in such a kit may contain a monoclonal antibody capable of binding to a sequence specific for a certain collagen type (for example, SEQ ID NOs: 11-13). Such an antibody may be provided in a form attached to any supporting material known to one skilled in the art (for example, wells in a microtiter plate, and a suitable membrane, such as nitrocellulose). Such a kit may further comprises components (for example, reagents or buffers) to be used in assays. Alternatively, such a kit may also be labeled with a substance as described above, which is suitable for direct or indirect detection of antibody binding.

The present invention is described below in more detail by way of examples. However, the present invention is not limited to the following examples. Unless otherwise specified, methods as described in Immunochemistry in Practice (Blackwell Scientific Publications) were used as the methods for preparing the antibodies. Also, unless otherwise specified, methods as described in Molecular Cloning : A Laboratory Manual, 2nd Edition (Cold Spring Harbor Laboratory) were used as the genetic engineering techniques.

### Example 1

### Generation of anti-collagen neoepitope antibody

### (1) Antigen immunization:

A hydroxyproline-containing neoepitope peptide represented by Gly -Pro-Hyp-Gly-Pro-Gln-Gly (SEQ ID NO: 2) was synthesized (Greiner Bio-one). A solution in which 10 mg of the synthesized neoepitope peptide is dissolved in 1 ml of 0.1 M phosphate buffer, pH 6.0, containing 5 mM EDTA was mixed with a solution in which 10 mg of giant keyhole limpet hemocyanin (maleimide KLH, PIERCE) was dissolved in 1 ml of purified water, and the mixture was allowed to react for 4 hours at room temperature and subsequently overnight at 4°C. The mixture was then dialyzed against distilled water and subsequently lyophilized, thereby obtaining 13 mg of a neoepitope peptide-KLH complex.
For the initial immunization, seven female A/J Jms Slc mice (4 week old) were intraperitoneally injected with 0.1 mg of the peptide-KLH complex in combination with complete Freund's adjuvant. At days 21, 42, and 63 after the initial immunization, the mice were boosted with 0.1 mg of the peptide-KLH complex in combination with complete Freund's adjuvant and further, at day 71, intraperitoneally injected with a solution in which 0.1 mg of the peptide-KLH complex is suspended in 0.1 ml of physiological saline, which was the final immunization.

### (2) Biotin labeling of the neoepitope peptide:

A solution in which 0.2 mg of the synthesized neoepitope peptide is dissolved in 0.4 ml of 0.1 M phosphate buffer, pH 6.0, containing 5 mM EDTA was mixed with a solution in which 0.60 mg of PEO-maleimide activated biotin (PIERCE) was dissolved in 0.1 ml of distilled water, and the mixture was allowed to react for 2 hours at room temperature and subsequently biotin-labeled neoepitope peptide was purified by reverse-phase HPLC.

### (3) Generation of hybridoma:

Three days after the final immunization, the spleens were removed to collect spleen cells. The spleen cells were fused with mouse myeloma cells (p3×63-Ag8.U1, Tokyo Oncology Institute) by using 50% polyethylene glycol 4000, and hybridomas were selected in medium containing hypoxanthine, aminopterin, and thymidine.

### (4) Selection of anti-neoepitope antibody:

Ten days after the cell fusion, screening for cells producing specific antibodies was conducted using ELISA as described below. To each well of a 384-well microtiter plate (Nunc), 35 µl of Tris buffer (50mM Tris-HCl, pH7.5) containing 0.35 µg of anti-mouse IgG antibody (Shibayagi) was added and incubated for 16 hours at 4°C for adsorption. Each well was washed once with 90 µl of washing solution (physiological saline containing 0.01% Tween20), and then 90 µl of Block Ace (Dainippon pharmaceuticals) was added. The plate was allowed to stand at room temperature for 2 hours for blocking (an anti-mouse IgG antibody-solid-phased plate). After each well was washed once with 90 µl of the washing solution, 10 µl of buffer A (50 mM Tris buffer, pH 7.4, containing 0.5% bovine serum albumin, 0.01% Tween 80, 0.05% Proclin 150 and 0.15 M NaCl) containing 15 µl of culture supernatant from hybridomas was mixed with 10 µl of buffer A containing 0.05 ng of the biotin-labeled neoepitope peptide and 2 ng of Streptavidin-HRP (PIERCE), and the mixture was allowed to react at 4°C for 16 hours.

Subsequently, each well was washed three times with 90 µl of the washing solution, and 25 µl of TMB-Substrate Chromogen (DAKO) was added and incubated for 30 minutes at room temperature for color development, followed by the addition of 25 µl of 0.05 M sulfuric acid to terminate the reaction. Then, the absorbance at 450 nm was measured.

From the results of the screening, 3 clones were selected from the 9 hybridoma clones reactive with the hydroxyproline-containing neoepitope peptide. These 3 clones also showed affinity to the neoepitope peptide with no hydroxyproline. From the clones obtained, one clone was selected that showed strong affinity to the neoepitope peptide, whether in the presence or absence of hydroxylation, and whose binding to the neoepitope peptide was inhibited by collagenase-digested type II collagen. This clone was designated 20A10. 20A10 was tested for isotype by using Mouse Immunoglobulin Isotyping ELISA Kit (BD Biosciences). The results showed that the isotype of 20A10 is IgG1/κ.

### Example 2

### Epitope analysis of the neoepitope antibody (20A10) using synthetic peptides

To investigate the specificity of the neoepitope antibody (20A10) forneoepitope recognition, neoepitope peptides having the following amino acid sequences were used according to the method described below.
Asp-Gly-Pro-Ser-Gly-Ala-Glu-Gly-Pro-HyP-Gly-Pro-Gln-Gly (corresponding to positions 962-975 of the amino acid sequence shown in SEQ ID NO: 20, SEQ ID NO: 14)
Gly-Pro-Gln-Gly (corresponding to positions 972-975, SEQ ID NO: 15)
Gly-Pro-Pro-Gly-Pro-Gln-Gly-Leu-Ala-Gly-Gln-Arg (corresponding to positions 969-980 of the amino acid sequence shown in SEQ ID NO: 20, SEQ ID NO: 16)
Gly-Glu-Pro-Gly-Asp-Asp-Gly-Pro-Ser-Gly-Ala-Glu-Gly-Pro-HyP-Gl y-Pro-Gln-Gly (corresponding to positions 957-975 of the amino acid sequence shown in SEQ ID NO: 20, SEQ ID NO: 17)
Gly-Glu-Pro-Gly-Asp-Asp-Gly-Pro-Ser-Gly-Ala-Glu-Gly-Pro-Pro-Gl y-Pro-Gln-Gly (corresponding to positions 957-975 of the amino acid sequence shown in SEQ ID NO: 20, SEQ ID NO: 18)

To a 96-well microtiter plate (Nunc), 150 µl of Tris buffer (50mM Tris-HCl, pH7.5) containing 1.5 µg of anti-mouse IgG antibody (Shibayagi) was added and incubated overnight at 4°C for adsorption. Each well was washed once with 0.3 ml of washing solution (physiological saline containing 0.01% Tween20), and then 0.3 ml of Block Ace (Dainippon pharmaceuticals) was added. The plate was allowed to stand at room temperature for 2 hours for blocking. (An anti-mouse IgG antibody-solid-phased plate). After each well was washed once with 0.3 ml of the washing solution, 50 µl of buffer A containing each of the above neoepitope peptides at a concentration of 0.001-125µM was mixed with 50. µl of buffer A containing 0.1 ng of the biotin-labeled neoepitope peptide (SEQ ID NO: 2) and 4 ng of Streptavidin-HRP and 50 µl of buffer A containing 0. 5 ng of the anti-neoepitope antibody (20A10), and the mixture was allowed to react at 4°C for 16 hours. Subsequently, each well was washed three times with 0.3 ml of the washing solution, and 0.1 ml of TMB-Substrate Chromogen was added and incubated for 30 minutes at room temperature for color development, followed by the addition of 0.1 ml of 0.05 M sulfuric acid to terminate the reaction. Then, the absorbance at 450 nm was measured.

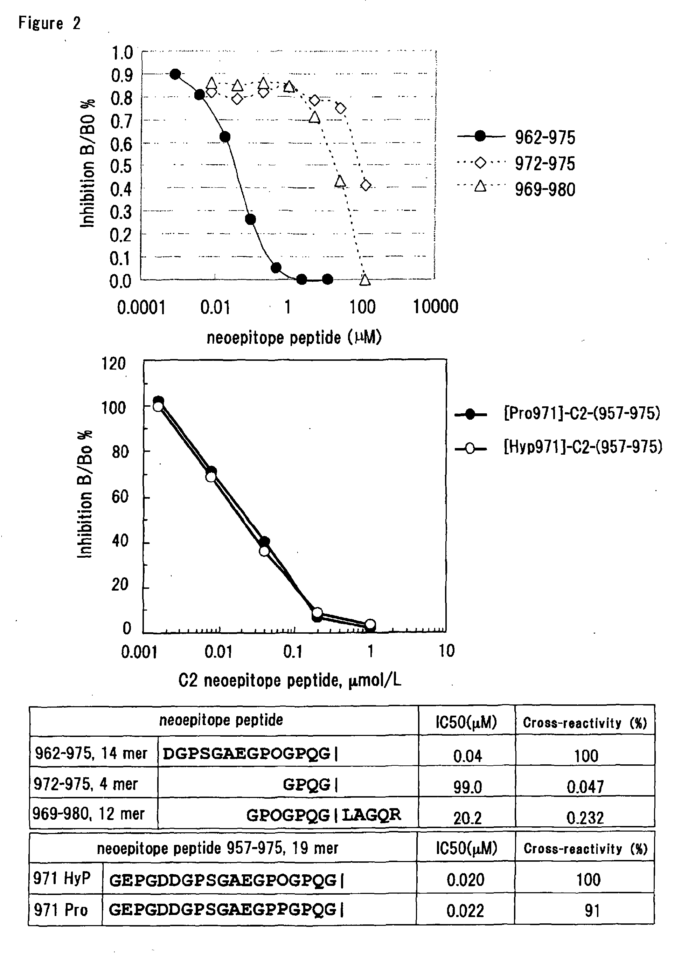

The results showed that the carboxy terminus of the glycine residue at position 975 of the neoepitope peptides is indispensable for binding with anti-neoepitope antibody 20A10, and that at least 5 residues upstream from the terminus are also indispensable (the graph shown in the upper part and the table in the lower part of Fig. 2).

Competitive immunoassays was conducted according to the method described above using a peptide consisting of 19 residues of the C-terminal neoepitope portion of type II collagen in which the residue at position 971 is hydroxyproline, and the same peptide, except that the residue at position 971 is proline. As a result, it was verified that the cross-reactivity of the non-hydroxylated form is 91%, and that hydroxylation of proline at position 5 from the terminus has little effect on the crossreactivity (the graph shown in the middle part and the table in the lower part of Fig. 2). Cases have been reported that the proline residue at position 971 was hydroxylated at a rate of 81% in human cartilage collagen. Also, it has been reported that anti-neoepitope antibody 9A4, reported in the prior art, has more than 90-fold lower affinity to hydroxylated proline located at the same position relative to non-hydroxylated proline(Downs JT et al., Journal of Immunological methods, 247: 25-34 (2001)). In contrast, 20A10 is capable of binding with equal affinity whether the residue is hydroxylated or not. Therefore, 20A10 has a high sensitivity of detecting a neoepitope fragment, and would allow for accurate quantification if the hydroxylation rate changes.

### Example 3

### Assessment of the specificity of the anti-neoepitope antibody (20A10) depending on the collagen types.

To 10 µg/10 µl of a solution of human type I, type II, or type III collagen (Chondrex), 10 µl of 2 x enzyme reaction buffer (50mM Tris buffer, pH 7.6, containing 0.3 M NaCl, 10 mM CaCl₂, and 0.005% Brij35) was added for neutralization. To the solution, 0.2 µg of activated human MMP13 (human Pro-MMP13 (Calbiochem), activated by incubation with 1 mM APMA at 37°C for 2 hours), and the mixture was allowed to react overnight at 37°C. Subsequently, stop solution (EDTA, final concentration 5 mM) was added to prepare the natural type neoepitope solution.

To a 384-well microtiter plate (Nunc), 35 µl of Tris buffer (50mM Tris-HCl, pH7.5) containing 0.35 µg of anti-mouse IgG-Fc antibody (Jackson Immuno Research) was added and incubated overnight at 4°C for adsorption. Each well was washed once with 90 µl of washing solution (physiological saline containing 0.01% Tween20), and then 0.1 ml of Block Ace (Dainippon pharmaceuticals) was added. The plate was allowed to stand for 2 hours at room temperature for blocking (An anti-mouse IgG antibody-solid-phased plate) After each well was washed once with 90 µl of the washing solution, 10 µl of buffer A containing 6.4-250 nM of the natural type neoepitope solution was mixed with 10 µl of buffer A containing 1 ng/ml of the biotin-labeled neoepitope peptide (SEQ ID NO: 2) and 200ng/ml of Streptavidin-HRP, and 10 µl of buffer A containing 15ng/ml of the anti-neoepitope antibody (20A10). Then the mixture was allowed to react at 4°C for 16 hours.

Subsequently, each well was washed three times with 90 µl of the washing solution, and 25 µl of TMB-Substrate Chromogen (DAKO) was added and incubated for 30 minutes at room temperature for color development, followed by the addition of 25 µl of 0.05 M sulfuric acid to terminate the reaction. Then, the absorbance at 450 nm was measured.

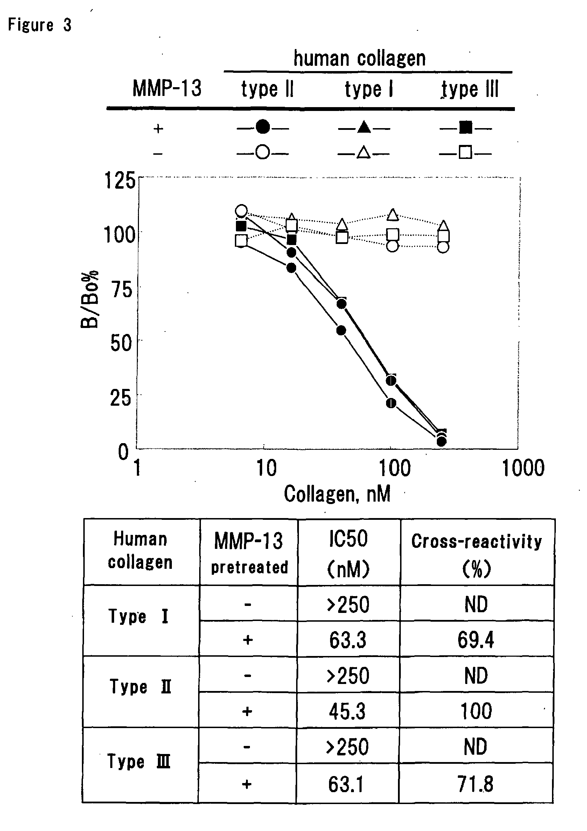

As a result, it was verified that while anti-neoepitope antibody 20A10 does not react with MMP13-undigested collagen, it specifically reacts with MMP13-digested collagen containing a terminal neoepitope. It was also verified that 20A10 is capable of binding to any neoepitopes of types I, II, and III collagens with equal affinity (Fig. 3). Accordingly, this antibody is capable of detecting a fragment of digested collagen with a high sensitivity, even in the presence of a large amount of undigested collagen, which do not form a background. Thus, it allows for accurate quantification of collagenase activity. It also allows for measurement of degradation of types I and III collagens, when combined, as a capture antibody, with an antibody capable of recognizing a specific site of type I or III collagen.

### Example 4

### Development of type II collagen-specific neoepitope measurement system

To measure type II collagen, a synthetic peptide of Gly-Glu-Pro-Gly-Asp-Asp-Gly-Pro-Ser (SEQ ID NO: 12), which corresponds to a portion of the type II collagen neoepitope (corresponding to positions 957-965 of the amino acid sequence shown in SEQ ID NO: 20) having a neoepitope in the C-terminal, was used as immunogen. To this peptide, a cysteine linker was added at the amino terminus and the carboxy terminus was amidated. A solution in which 2.1 mg of this synthetic peptide is dissolved in 1 ml of 0.1 M phosphate buffer (pH 6.0) containing 5 mM EDTA was mixed with a solution in which 8 mg of giant keyhole limpet hemocyanin (maleimide KLH, PIERCE) was dissolved in 3 ml of 0.1 M phosphate buffer (pH 6.0) containing 5 mM EDTA, and the mixture was allowed to react for 3 hours at room temperature.

The mixture was then dialyzed against distilled water and subsequently lyophilized, thereby obtaining 8 mg of a type II collagen-specific internal sequence peptide-KLH complex. For the initial immunization, each four female A/J Jms Slc and Balb/c mice (4 week old) were intraperitoneally injected with 0.04 mg of the peptide-KLH complex in combination with complete Freund's adjuvant.
At days 21, 42, and 63 after the initial immunization, the mice were boosted with 0.1 mg of the peptide-KLH complex in combination with complete Freund's adjuvant and further, at day 71, intraperitoneally injected with a solution in which 0.1 mg of the peptide-KLH complex is suspended in 0.1 ml of physiological saline. This was the final immunization. A solution in which 0.2 mg of the synthesized peptide is dissolved in 0.1 ml of 0.1 M phosphate buffer (pH 6.0) containing 5 mM EDTA was mixed with a solution in which 0.25 mg of HPDP-biotin (PIERCE) was dissolved in 0.1 ml of dimethylformamide, and the mixture was allowed to react overnight at 4°C, followed by the purification of biotin-labeled peptide by reverse-phase HPLC. Three days after the final immunization, the spleens were removed to collect spleen cells.

The spleen cells were fused with mouse myeloma cells (p3×63-Ag8.U1, Tokyo Oncology Institute) by using 50% polyethylene glycol 4000, and hybridomas were selected in medium containing hypoxanthine, aminopterin, and thymidine. Ten days after the cell fusion, screening for cells producing specific antibodies was conducted using ELISA as described below. To each well of a 384-well microtiter plate (Nunc), 35 µl of Tris buffer (50 mM Tris-HCl, pH 7.5) containing 0.35 µg of anti-mouse IgG antibody (Shibayagi) was added and incubated for 16 hours at 4°C for adsorption. Each well was washed once with 90 µl of washing solution (physiological saline containing 0.01% Tween20), and then 90 µl of Block Ace (Dainippon pharmaceuticals) was added. The plate was allowed to stand at room temperature for 2 hours for blocking (an anti-mouse IgG antibody-solid-phased plate). After each well was washed once with 90 µl of the washing solution, 10 µl of buffer A (50 mM Tris buffer, pH 7.4, containing 0.5% bovine serum albumin, 0.01% Tween 80, 0.05% Proclin 150 and 0.15 M NaCl, pH 7.4) containing 15 µl of hybridoma culture supernatant was mixed with 10 µl of buffer A containing 0.05 ng of the biotin-labeled neoepitope peptide and 2 ng of Streptavidin-HRP (PIERCE), and the mixture was allowed to react at 4°C for 16 hours.

Subsequently, each well was washed three times with 90 µl of the washing solution, and 25 µl of TMB-Substrate Chromogen (DAKO) was added and incubated for 30 minutes at room temperature for color development, followed by the addition of 25 µl of 0.05 M sulfuric acid to terminate the reaction. Then, the absorbance at 450 nm was measured. From the results of the screening, 4 hybridoma clones were selected that showed strong affinity to the type II collagen immunogen peptide (SEQ ID NO: 12) but not react with the other types of collagen. From the clones obtained, one clone was selected that reacts with natural neoepitope of type II collagen but not with that of type I or III collagen. This clone was designated 6G4. 6G4 did not react with native type II collagen but reacted with collagenase-digested type II collagen

### Development of type II collagen neoepitope quantitative measurement system by sandwich ELISA:

A synthetic peptide Gly-Glu-Lys-Gly-Glu-Pro-Gly-Asp-Asp-Gly-Pro-Ser-Gly-Ala-Glu-Gly-Pro-Hyp-Gly-Pro-Gln-Gly (corresponding to positions 954-975 (SEQ ID NO: 19)), which consists of 22 residues comprising the neoepitope and collagen type-specific internal sequence, was synthesized as a calibration standard peptide. HRP-labeled 20A10 was prepared. More specifically, 0.05 ml of 0.1 M mercaptoethylamine solution was added to 0.5 ml of 5 mM EDTA-containing 0.1 M phosphate buffer (pH 6.0) containing 1 mg of the IgG fraction of 20A10, and the mixture was allowed to react at 37°C for 1.5 hours and then subjected to gel filtration with a PD-10 column (GE Healthcare) to fractionate the reduced IgG fraction. Sulfo-SMCC (PIERCE) was added to 0.2 ml of 5 mM EDTA-containing 0.1 M phosphate buffer (pH 6.0) containing 1 mg of peroxidase (derived from Horse radish, Roche: HRP) , and the mixture was allowed to react for 2 hours at room temperature and then subjected to gel filtration with a PD-10 column (GE Healthcare) to fractionate the maleimide HRP fraction. To this fraction, the reduced IgG fraction of 20A10 was added, and the mixture was allowed to react overnight at 4°C, and then subjected to high-speed gel filtration (LC-6A system (Shimadzu) attached with a TSK-GEL G3000 column (Tosoh) equilibrated with 5 mM EDTA-containing 0.1 M phosphate buffer; pH 6.0), thereby fractionating about 0.5 mg of the HRP-labeled 20A10 fraction. To each well of a 96-well microtiter plate (Nunc), 150 µl of Tris buffer (50 mM Tris-HCl, pH 7.5) containing 1.5 µg of type II collagen internal sequence-specific antibody 6G4 was added and incubated for 16 hours at 4°C for adsorption. Each well was washed once with 300 µl of washing solution (physiological saline containing 0.01% Tween20), and then 150 µl of Block Ace (Dainippon pharmaceuticals) was added. The plate was allowed to stand at room temperature for 2 hours for blocking. After each well was washed once with 300 µl of the washing solution, 50 µl of buffer A (50 mM Tris buffer, pH 7.4, containing 0.5% bovine serum albumin, 0.01% Tween 80, 0.05% Proclin 150 and 0.15 M NaCl, pH 7.4) containing 10-500 pM standard peptide or a test sample was mixed with 100 µl of buffer A containing 0.05 ng of HRP-labeled anti-neoepitope antibody 20A10, and the mixture was allowed to react at 4°C for 16 hours. Subsequently, each well was washed three times with 300 µl of the washing solution, and 100 µl of TMB-Substrate Chromogen (DAKO) was added and incubated for 30 minutes at room temperature for color development, followed by the addition of 100 µl of 0.05 M sulfuric acid to terminate the reaction. Then, the absorbance at 450 nm was measured.

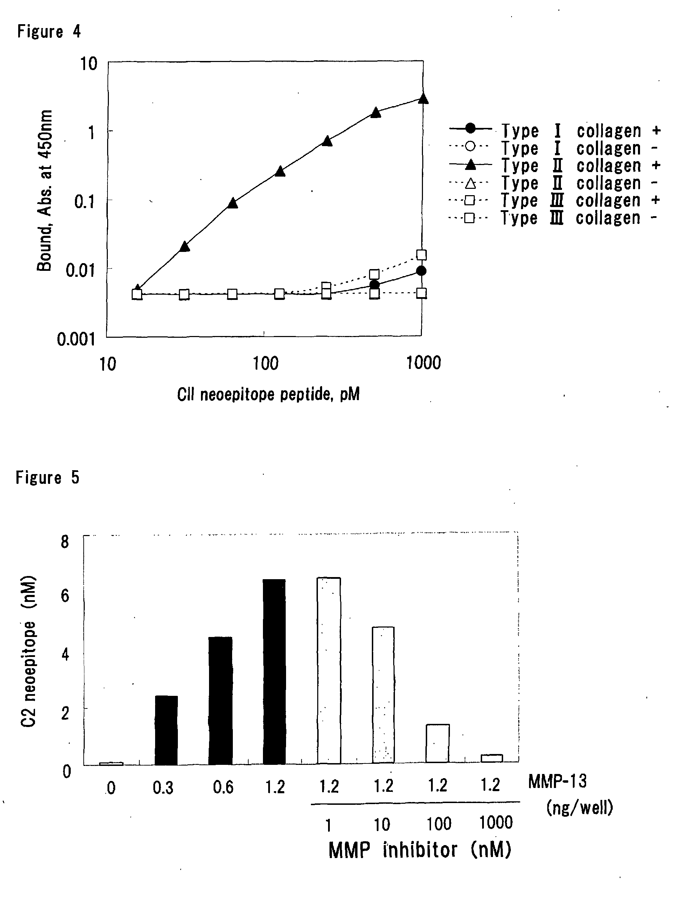

As a result, the antibody reacted only with the collagenase-digested type II collagen, and its lower detection limit was 10 pM (Fig. 4). Unlike the above anti-neoepitope antibody 9A4, which shows low affinity to the hydroxylated forms, antibody 20A10 is capable of binding to both of the non-hydroxylated and hydroxylated forms with equal affinity. Therefore, conversion of the measured values to the proline/hydroxyproline ratio is not necessary, and accurate quantification of the neoepitope concentration is possible without being influenced by the hydroxylation.

### Example 5

### In vitro collagenase activity measurement system

Five ng/ml of a solution of human type II collagen (Chondrex) was added to a 96-well MaxiSorp plate (NUNC). The plate was incubated overnight at 4°C, and washed twice with washing buffer (0.05 M Tris-HCl, pH 7.6), thereby providing a collagen-coated plate. To a preincubation plate (Costar), enzyme-reaction buffer (50mM Tris buffer, pH 7.6, containing 0.3 M NaCl, 10 mM CaCl₂, 0.005% Brij35), activated human MMP13, a member of human type II collagenase, and an MMP inhibitor, and then incubated for 30 minutes at room temperature, followed by the measurement of the amount of the collagen neoepitope present in the enzyme-reaction buffer using the sandwich ELISA system described in Example 4.

The results showed that the measurements of the neoepitope increased in a manner dependent on the dose of MMP13 added while the neoepitope production by the MMP13 was inhibited in a manner dependent on the dose of the MMP inhibitor (Fig. 5).

### Example 6

### Collagenase activity measurement system in human chondrocyte culture system

Ten ng/ml of a solution of human type II collagen is added to a 96-well culture plate (Sumitomo Bakelite). The plate is incubated overnight at 4°C, and washed once with culture medium (DMEM medium containing 0.1 mg/ml BSA, ITS and 50 µM L-ascorbic acid), thereby providing a collagen-coated plate.
Normal human-derived chondrocytes (Chondrex) are seeded into the coated plate at a density of 4 x 10⁴ per well and incubated with the culture medium at 37°C in a 5% CO₂ atmosphere. One day after, the culture medium is replaced, and 1 ng/ml human interleukin 1β (Genzyme), 10 ng/ml Oncostatin M (Sigma), and a test MMP inhibitor are added at various concentrations. The cells are cultured further for 2 days. Four days after, after a stop solution (EDTA, final concentration 5 mM) is added, the culture supernatant is collected. The activity of the MMP inhibitor was determined by measuring the concentration of the collagen neoepitope present in the supernatant using the sandwich ELISA system described in Example 4.

As a result, it is verified that the MMP expression is induced in the chondrocytes by IL-1β stimulation and degradation of type II collagen is enhanced, and that the MMP inhibitor inhibits the collagen degradation in the chondrocytes in a dose-dependent manner (Fig. 6). These results demonstrate that this measurement system is useful for assessment of test MMP inhibitors.

### Example 7

### Amino acid sequence analysis of 20A10

RNA was extracted from the established hybridoma cells, using RNeasy Mini Kit (QIAGE). DNA fragments were amplified from 1 µg of the extracted RNA by using 5' RACE Syatem for Rapid Amplification of cDNA Ends, Version 2.0 (Invitrogen) . The amplified fragments were cloned using TOPO TA Cloning Kit (Invitrogen) and their nucleotide sequences were analyzed using Applied Biosystems 3130 Genetic Analyzer (Applied Biosystems). As a result, the amino acid sequences of the variable regions were specified (Fig. 7).

### Industrial Applicability

The present invention allows for accurate detection and quantification of types I, II, and III collagen neoepitopes by type, without being affected by hydroxylation of proline, which is altered depending on the physical/nutritional conditions. In particular, cleavage of type II collagen by collagenase is a potential indicator of cartilage metabolism and is useful for a diagnostic method or kit for assessing the progression of diseases or therapeutic effects in cartilage diseases such as osteoarthritis. In addition, cleavage of type I collagen by collagenase is a potential indicator of extracellular matrix metabolism in the connective tissues throughout the body and for and is useful for a diagnostic method or kit for assessing the progression of fibrosis in various organs and the effect of anti-fibrosis therapy.

## Claims

1. A monoclonal antibody specifically binding to a collagen neoepitope fragment at positions 962 to 975 of the amino acid sequence shown in SEQ ID NO: 20, wherein the binding affinity is substantially the same whether proline contained in said epitope is in a non-hydroxylated form or in a hydroxylated form.

2. The monoclonal antibody of claim 1, wherein the proline of claim 1 is located at position 971 of the amino acid sequence shown in SEQ ID NO: 20.

3. The monoclonal antibody of claim 1, wherein the epitope is located in the region of positions 957 to 975 of the amino acid sequence shown in SEQ ID NO: 20.

4. The monoclonal antibody of claim 2, wherein in an immunoassay using the peptide consisting of the amino acid sequence shown in SEQ ID NO: 14, 17, or 18 as the competitor to inhibit the immunoreaction of said antibody with the peptide consisting of the amino acid sequence shown in SEQ ID NO: 2, 50% inhibitory concentration for the immunoreaction is 0.04 µM or lower for either of the peptides.

5. A monoclonal antibody having:
1) in a complementarity-determining region, a heavy chain variable region containing the following amino acid sequences: KYGIN (SEQ ID NO: 5), WINTYSGMTT YADDFKG (SEQ ID NO: 6), and SLGYDYGGFAY (SEQ ID NO: 7); and
2) in a complementarity-determining region, a light chain variable region containing the following amino acid sequences: RSGQTLVHDNENTYFH (SEQ ID NO: 8), KISNRFS (SEQ ID NO: 9), and SQNTHVPFT (SEQ ID NO: 10).

6. A monoclonal antibody having:
1) a heavy chain variable region containing the amino acid sequence of SEQ ID NO: 3; and
2) a light chain variable region containing the amino acid sequence of SEQ ID NO: 4.

7. The monoclonal antibody of any one of claims 1-6, which is labeled.

8. An immunoassay using the monoclonal antibody of any one of claims 1-6.

9. A method for measuring the amount of a collagen neoepitope fragment, using the monoclonal antibody of any one of claims 1-6.

10. A method for measuring collagenase activity, wherein the amount of a collagen neoepitope fragment measured using the monoclonal antibody of any one of claims 1-6 is used as an indicator.

11. A method for screening for a collagenase inhibitor, wherein the amount of a collagen neoepitope fragment measured using the monoclonal antibody of any one of claims 1-6 is used as an indicator.

12. A method for selecting patients with collagenase related diseases, comprising a step of measuring the amount of a collagen neoepitope fragment contained in a biological sample, by using the monoclonal antibody of any one of claims 1-6.

13. A kit comprising the monoclonal antibody of any one of claims 1-6.

14. A method for diagnosing collagenase-related diseases, comprising a step of measuring the amount of a collagen neoepitope fragment contained in a biological sample, by using the monoclonal antibody of any one of claims 1-6.

15. The monoclonal antibody of any one of claims 1-6 for use in diagnosis of collagenase-related diseases.
